# EUROPEAN PATENT SPECIFICATION

(11) **EP 3 661 461 B1**
(45) Date of publication and mention of the grant of the patent: **11.11.2026**
(21) Application number: 18745979.7
(22) Date of filing: 02.08.2018
(51) Int. Cl.: A61F 2/36, A61F 2/32, A61F 2/46, A61F 2/30

(54) **CUSTOM MADE HIP IMPLANT**
MASSGESCHNEIDERTES HÜFTIMPLANTAT
IMPLANT DE HANCHE PERSONNALISÉ

(30) Priority: 02.08.2017 EP 17184441
(43) Date of publication of application: 10.06.2020
(73) Proprietor: Bispebjerg Hospital, 2400 Copenhagen NV (DK)
(72) Inventor: LAURITZEN, BRUUN, Jes, 1270 København K (DK)
(74) Representative: Høiberg P/S
(86) International application number: PCT/EP2018/071003
(87) International publication number: WO 2019/025546

(56) References cited:
- WO-A1-2014/059388
- WO-A2-2014/094785
- US-A1- 2009 226 068
- US-A1- 2013 211 529

## Description

The present disclosure relates to a custom made implant for reducing the pain for subjects with diseased hip joints. The present disclosure further relates to a system and method for determining the most suitable hip joint treatment for a subject and for determining subject eligibility for receiving the presently disclosed customized implant.

### Background

Hip replacement surgery is one of the most common orthopaedic operations performed today. Typical reasons for hip replacement surgery include osteoarthritis, avascular necrosis and hip fractures. The surgery is performed for relieving pain for the subject and for improving hip function.

Hip surgery is normally carried out as total hip replacement in which the entire femoral head is removed and a stem with an artificial femoral head is inserted into the femur. Total hip replacement also includes inserting a shell into the acetabulum, although this can be omitted in some cases. The implant is either cemented in place or inserted without using cement in cases where the bone quality is good. Both methods may offer clinically good results, but long-term loosening of the implants still remains a challenge and may require major revision surgery.

Although hip replacement surgery is a well-known procedure, problems with this type of surgery persist. The surgery is extensive regarding soft tissue, but also requires bone resection of the femoral head and reaming of the acetabulum. When inserting and impacting the implant into the femur, there is a risk of producing periprosthetic fractures in the bone. Furthermore, the invasive character of the surgery from cutting the bone and fixating the implant using e.g. cement increases the risk of infection in the area. Again, there is a significant risk of complications due to loosening of the implant over time.

Total hip replacements may have plastic/polyethylene components or may be metal-on-metal implants. This introduces the risk that the implant gives off small particles during use due to abrasion caused by friction from movement of the hip joint. Galvanic and crevice corrosion may also occur in such situations.

The list of problems mentioned above signals the need for a less invasive medical implant for hip surgery which brings fewer complications. An implant in the form of a dome shaped shell attached to the femoral head has previously been put forward by the same inventor as for the present disclosure, cf. WO 2014/094785. The patent application describes a shell for attachment to the femoral head. Such implants are produced in certain predetermined sizes and the implant best suited for the subject is chosen. This has mitigated some of the problems, e.g. a significantly lesser extensive surgery is needed.

US2009/226068 A1 regards implants for altering wear patterns of articular surfaces.

US 2013/211529 A1 discloses a joint augmentation implant according to the preamble of claim 1.

### Summary

The invention is defined by the claims.

The purpose of the present disclosure is to provide a hip implant which requires a less invasive surgical procedure than the common total hip replacement.

The problems mentioned above are remedied by the medical implant as described in claim 1.

The presently disclosed implant preferably consists of only a single part and is therefore simpler than implants for total hip replacement. The simpler surgical procedure involved in implanting the presently disclosed customized implant compared to the prior art (and more complicated) implants for total hip replacement also leads to reduced cost in relation to the surgery. Implants used for total hip replacement do not necessarily need patient specific customization, and such implants exist in a fixed scale of sizes.

The presently disclosed implant is customized in order to fit exactly to the patient needing the implant. As stated above the customization can be based on at least one 3D computed tomography image such that the entire surface of the femoral head and acetabulum may be used for determining the suitable parameter(s) needed for customizing the implant.

It may be advantageous to utilize an implant with varying thickness. Patients eligible for the customized hip implant may also suffer from leg length discrepancy, which can be caused by the diseased joint. Therefore, in an embodiment of the disclosure the thickness tₜ at the top of the dome is different from the equatorial thickness. For the case where the patient's leg having a diseased hip is shorter than the other leg, the implant is customized such that the thickness at the top of the implant is larger than the equatorial thickness which may thereby at least partly remedy the leg length discrepancy. The thickness of the implant is in one embodiment selected based on a radiographic measurement of the leg length discrepancy.

Preferably the present implant has smooth inner and outer surfaces which reduces and nearly eliminates the friction in the hip joint. Because the implant is constructed with smooth inner and outer surfaces, it is possible that the implant is at least initially unconstrained and can move against both the femoral head and the acetabulum. Therefore, the presently disclosed implant is preferably configured for at least initially unconstrained attachment to the genuine diseased femoral head. This means that the femoral head can move freely against the smooth inner surface of the implant and the acetabulum can move freely against the smooth outer surface of the implant. Thereby, the friction in the joint may be further reduced and can relieve the pain even more.

The initially unconstrained implant may eventually become attached to the femoral head or the acetabulum. This may occur by natural tissue growth near or around the implant. In such cases, the implant will likely attach to the part with most friction and leave the part with less friction free for movement of the joint. The smooth surface of the unattached part of the implant will thereby still provide low friction in the joint and still relieve the pain. Because the implant is initially free to move against both the femoral head and acetabulum, the implant may eventually become attached to the femoral head or acetabulum in the naturally most favourable configuration.

Because the implant consists of a single part, problems with corrosion and particles being released from the implant are significantly reduced. This is because the implant only contacts the original bone of the joint and no metal-on-metal contact is present.

The present disclosure brings other advantages compared to known methods. The procedure is much less invasive, and the much smaller size of the customized hip implant than e.g. implants for full hip replacement allows for the surgery to be carried out with a smaller incision in the patient, which means there is less soft tissue damage and lower blood loss during surgery. The implant is designed to require no or very little bone removal during surgery. This again means a much less invasive procedure and reduces the risk of periprosthetic fractures forming near the implant. Because the surgery is less invasive it also requires a shorter hospital stay and the patient recovers faster after the surgery. Additionally, no cement is needed for the implant, meaning that there is no risk of the patient having an allergic reaction to cement.

In order for the implant to fit to the femoral head, the dimensions of the implant must be customized. Additionally, the femoral head needs to have a sufficiently high sphericity, i.e. the femoral head should preferably be close to spherical for a good result when attaching the implant to the femoral head. During surgery and insertion of the implant the soft tissues, capsule and ligament may be sacrificed to create place for the final implant.

The presently disclosed implant can be used in humans, but may also be constructed to fit animals e.g. dogs and horses. In such cases the dimensions of the implant needs to be adjusted to fit the joint of the animal.

In order for the presently disclosed customized hip implant to successfully alleviate the patient's pain from a degenerated osteoarthritic cartilage surface, the bone of the femoral head often needs to be sufficiently preserved. Therefore, the present disclosure further relates to a method for determining the most suitable hip joint treatment for a subject. The first step of the method comprises obtaining at least one 3D computed tomography image showing substantially the entirety of the femoral head and the acetabulum of a hip joint of said subject. The second step involves extracting the shape of the femoral head and/or the acetabulum from said at least one 3D computed tomography image. Subsequently the shapes of the femoral head and/or the acetabulum are evaluated to determine the most suitable treatment.

The shapes of the bones in the joint may generally be extracted from a regular scan showing the hip joint of the subject. However, in some cases the bone material of the joint may be very close together, thereby making it more difficult to separate and extract the shapes of the bones in the scan. In circumstances where the extracted shapes are questionable and the quality may not be satisfactory, it may be considered applying a force to the leg to better separate the bones in the scan. Therefore, in one embodiment of the presently disclosed method the 3D computed tomography image of the hip is performed with traction applied to the leg which may create enough separation in the joint during the scan for discerning the bones such that the joint can be analyzed.

When at least one 3D computed tomography image is obtained, the image can be analyzed in order to determine the most suited treatment for the diseased hip joint. In case the medical implant of the present disclosure is the most suited treatment, the customization parameters for the implant need to be determined. A conversion of the 3D computed tomography image of bones in the joint may illustrate the potential shape of the implant. This conversion may include one or more of inversion of the image, interpolation, rotation, segmentation, translation and other adjustments for completing the analysis. This analysis allows the joint to be digitally separated and modified in order to optimize the fit and dimensions of the implant. The 3D computed tomography image may further be used for creating a digital template of the femoral head, the acetabulum and a trial implant in order to check the fit of the implant to the joint.

The present disclosure further relates to a decision support system for assessing eligibility of a subject for the customized medical implant and/or for selecting the customization parameters for the customized medical implant, given at least one 3D computed tomography image showing substantially the entirety of the femoral head and the acetabulum of a hip joint of said subject. The system comprises a processing unit configured for extracting the shapes of the femoral head and acetabulum from said at least one 3D computed tomography image. The processing unit is further configured for evaluating the shapes of the femoral head and acetabulum extracted from said at least one 3D computed tomography image to determine subject eligibility. The system may be configured for selecting one or more parameters to be customized and for determining the value of said one or more parameter(s). The customizable parameters include, but are not limited to; a height h, an inner equatorial shell radius rₛ, an orifice radius rₒ, a thickness tₛ at the equatorial line, and a thickness tₜ at the top of the dome,

### Description of drawings

- **Fig. 1**: shows a cross-sectional side view of one embodiment of the customized medical implant for attachment to the femoral head.
- **Fig. 2**: is an example of a 3D computed tomography image of the pelvic area of a patient used for the presently disclosed disclosure.
- **Fig. 3**: shows an example of a 3D computed tomography image where the femoral bone part has been isolated.
- **Fig. 4**: shows an example of a 2D X-ray image anterior-posterior projection of both hip joints. The X-ray image is based on a computerized tomography scanning. The femoral heads are encircled by digital means.
- **Fig. 5**: shows an example of a 2D X-ray horizontal plane of the right hip joint. The X-ray image is based on a computerized tomography scanning. The femoral head is encircled by digital means.
- **Fig. 6**: is an example of a CT scan of both hip joints in anterior-posterior plane. Both the femoral head and the acetabulum are encircled by digital means. The diameters of the circles are also shown, which may be used as an indicator for the thickness of the potential implant.
- **Figs. 7A-D**: are screenshots from ITK-SNAP used in one embodiment for segmentation of the scan and identification of bones and surfaces. Here, only the program with its built-in functions was used for segmentation.
- **Figs. 8A-D**: are screenshots from ITK-SNAP used in another embodiment for segmentation of the scan and identification of bones and surfaces. Some points have been created and/or adjusted manually to create a better model of the joint.
- **Fig. 9**: shows a surface representation of the pelvic bone from a scan. The raw scan shown on the right contains many edges and steps. A marching cubes algorithm together with a surface reconstruction algorithm is used to produce the smooth surface shown on the left.
- **Fig. 10**: is an illustration of the scanned femoral bone (left), points on the surface of the bone used for determining implant dimensions (middle) and the bone surface with a sphere fitted to the femoral head (right).
- **Fig. 11**: shows a visualization of the scanned bones of a hip joint and an implant calculated and customized to fit the specific joint.

### Detailed description

As mentioned earlier the implant needs to be customized in order to perfectly fit the diseased hip of the patient. Therefore, the shell is preferably constructed such that one or more of the thickness at the equatorial line tₛ, the thickness at the top tₜ, the equatorial shell radius rₛ, the orifice radius rₒ and the shell height h are selected by fitting a sphere to the femoral head in said 3D computed tomography image. The shell may furthermore be constructed such that one or more of the thickness tₛ, the thickness tₜ, the equatorial shell radius rₛ, the orifice radius rₒ and the shell height h are selected by fitting a sphere to the acetabulum in said 3D computed tomography image. The implant should be constructed such that the implant fits between the femoral head and acetabulum in the best way possible such that pain and discomfort is relieved as much as possible. The parameters may also be determined from slices of a 3D computed tomography image, from x-ray images of the hip or alternatively by using trial and error fitting of the implant in a computer model of the hip. Furthermore, the parameters may be determined by fitting circles to 2D images of the hip joint.

Preferably the implant is constructed such that the equatorial shell radius is larger than the orifice radius rₛ > rₒ, and the height is larger than the equatorial shell radius h > rₛ. The orifice radius should fit the radius of the femoral head such that the implant can be pushed onto the femoral head. Selecting the orifice diameter to match the largest diameter of the femoral head means that the implant can be attached without risking damaging the femoral head. Cartilage and other soft tissue extending to and below the equatorial region of the femoral head may deform when the implant is attached and may reduce the risk of the implant becoming detached from the femoral head. In one embodiment the orifice is circular and defined by a circumferential rounded edge. This will make it easier to force the implant onto the femoral head and reduce the risk of damaging the bone in the process. Although the implant may be forced onto the femoral head it may still be customized such that it can move relative to the femoral head. Therefore, in another embodiment the medical implant is configured for at least initial unconstrained attachment to the genuine diseased femoral head.

In order for the implant to move efficiently relative to the acetabulum and with as little friction as possible, the outer surface of the shell is spherical at least above the equatorial plane. More preferably the entire outer surface of the shell is spherical. The shell is preferably unconstrained at least initially after implanted in the hip. The inner surface of the shell is spherical at least above the equatorial plane. More preferably the entire inner surface of the shell is spherical. Having spherical inner and outer surfaces allows the implant to freely rotate and tilt relative to both the femoral head and the acetabulum and reduces the risk of implant impingement at the acetabular rim. As a further means for reducing the friction, the implant is in one embodiment constructed such that the inner surface and/or the outer surface of the shell are smooth and preferably polished to obtain a surface roughness less than 0.1 mm.

In yet another embodiment the inner and outer surfaces of the shell are spherical, but where the radius of curvature of the inner surface of the shell is less than the radius of curvature of the outer surface of the shell such that the thickness of the shell at the top of the dome is larger than the thickness of the shell at the equatorial line tₜ > tₛ. However, selecting a larger thickness of the shell at the top than at the equator of the shell is used to compensate leg length discrepancy. Therefore, in yet another embodiment the thickness tₜ at the top of the dome is selected based on a radiographic measurement of the leg length discrepancy such that leg length discrepancy can be corrected or reduced. This is preferred when the customization of the implant allows for increased thickness at the top of the shell and when the patient's leg having a diseased hip is shorter than the other leg.

The thickness of the shell should be selected to be as large as possible based on the 3D computed tomography image of the joint. This will increase the strength of the implant such that the risk of damage or deformation is reduced and will make the implant more durable. In one embodiment of the disclosure the minimum thickness of the shell is selected to be at least 0.6 mm, or at least 0.75 mm, or at least 1.0 mm, or at least 1.2 mm, or at least 1.5 mm, or at least 1.8 mm, or at least 2.0 mm, or at least 2.5 mm. The minimum thickness of the shell may also depend on the size of the joint it is inserted into. In some embodiments the thickness of the shell is at least 1.0 mm for humans and at least 0.75 mm for dogs. In another embodiment the edge at the orifice of the shell is rounded such that the radius of curvature is half of the thickness of the shell at the orifice. This means that there are no sharp edges on the implant that could potentially damage the bone during attachment of the implant to the femoral head or after surgery.

As mentioned earlier, the implant is constructed such that the thickness of the shell varies in order to reduce or correct for leg length discrepancy. This is achieved by displacing the inner surface of the implant compared to the outer surface, such that they do not share a common center.The inner surface is displaced downward (towards the orifice) by some amount, thereby increasing the thickness at the top of the implant.

In order for the medical implant to be attachable to the femoral head, the orifice should be large enough for the implant to be forced onto the femoral head. Therefore, in one embodiment the orifice radius rₒ is selected such that rₒ corresponds to or is larger than the maximum diameter of the bone material of the genuine femoral head in said 3D computed tomography image. The implant is preferably constructed such that the height of the shell is greater than the equatorial shell radius such that the rounded or spherical shape of the shell extends below the equatorial plane of the shell, thereby allowing better movement of the joint. In another embodiment the ratio between the height and equatorial shell radius h/rₛ is therefore selected to be at least 1.24, or at least 1.27, or at least 1.30, or at least 1.32, or at least 1.35, or at least 1.38.

Alternatively the ratio between the height and equatorial shell radius h/rₛ is selected to be less than 1.40, or less than 1.35, or less than 1.32, or less than 1.30, or less than 1.27, or less than 1.24. Because the orifice is equal to or larger than the femoral head and the height of the shell is larger than the equatorial shell radius, the femoral head will be slightly smaller than the equatorial shell radius. This should not cause complications as it allows the implant to be initially unconstrained and move relative to the femoral head. Furthermore, cartilage and other soft tissue extending to and below the equatorial area of the femoral head may reversibly deform when the implant is forced over the femoral head and then at least partly return to the original shape. This will help ensuring that the implant is firmly attached and reduces the risk of the implant detaching from the joint. Over time the implant may become attached to either the femoral head or the acetabulum which will occur by natural tissue forming around the implant.

In a preferred embodiment, the outer equatorial shell radius is determined from the radius of a circle or sphere that fits in the acetabulum in said at least one 3D computed tomography image. Matching the equatorial shell radius with the radius of a circle or sphere that fits in the acetabulum ensures a good fit between the implant and the acetabulum. Thereby, pain and discomfort from the implant are reduced.

The selected orifice radius, outer equatorial radius and shell thickness based on the at least one 3D computed tomography image are preferably used for determining the parameters needed in order to construct the implant. In one embodiment the height may also be determined from the orifice radius, the outer equatorial shell radius and the thickness of the shell. Additionally, a possible measured difference in leg length may be used for determining if and how much the thickness of the shell should vary.

In some cases the femoral head may not be close to spherical and may e.g. have a large diameter in one direction and a smaller diameter in another direction. It may then be advantageous to deform the implant in a reversible manner prior to attachment to the femoral head such that the implant becomes at least partly elliptical. The implant can then be forced onto the femoral head in its deformed state and then return at least partly to its original shape.

The material used for fabricating the shell may be a metal or an alloy. In a preferred embodiment the material for the shell is a cobalt chromium molybdenum alloy such as the Co28Cr6Mo alloy such as the Wrought (UNS R31537, UNS R31538 or UNS R31539) alloys. In another embodiment the material for the shell is a steel alloy such as 316LVM, or a titanium alloy such as Ti6Al4V.

The present disclosure is furthermore related to a method for determining the most suitable hip joint treatment for a subject, the method is however not forming part of the present invention. This method includes the step of obtaining at least one 3D computed tomography image of the diseased hip. In some cases the two parts of the joint may appear too close together in the 3D computed tomography image. This may be caused by damaged or missing cartilage in the joint. In such cases it is difficult to separate the femoral head and the acetabulum for customization of the implant. Therefore, in one embodiment of the disclosure the at least one 3D computed tomography image of the hip is performed with traction applied to the leg. Joint traction may improve the 3D computed tomography such that the femoral and acetabular bones become discernable in cases where it is otherwise problematic. The traction force applied to the leg may in some embodiment be at least 10 kg or about 100 N. Traction may be applied using a traction brace that applies force between the pelvis and the leg e.g. the thigh, the calf or preferably the foot. The traction brace should preferably be constructed from non-metallic materials in order to not interfere with the 3D computed tomography scan. In another embodiment an x-ray fluoroscopy image of the hip of the subject is used to determine if traction to the leg is necessary during 3D computed tomography imaging of the hip.

The femoral head and the acetabulum should preferably be sufficiently preserved for the implant to alleviate the pain as much as possible. The shapes of the femoral head and acetabulum obtained from the at least one 3D computed tomography are evaluated in order to assess patient eligibility. Therefore, in one embodiment of the method the evaluation of the shape of the femoral head is based on the roundness of the femoral head in at least one cross-sectional scan. In another embodiment the evaluation of the shape of the acetabulum is based on the roundness of the acetabulum in at least one cross-sectional scan. In yet another embodiment the roundness should have a tolerance zone of at least 1 mm, or at least 2 mm, or at least 3 mm, or at least 4 mm for the patient to be eligible for the medical implant. The tolerance zone means that e.g. the femoral head in a cross-sectional scan should fit within two circles having a difference in radii equal to the tolerance. In yet another embodiment the roundness should be at least 0.70, or at least 0.80, or at least 0.85, or at least 0.90, or at least 0.93, or at least 0.96 for the patient to be eligible for the medical implant.

The roundness is preferably determined from a 2D cross-sectional scan of the femoral head or acetabulum. However, the evaluation may also be based on the sphericity, which is preferably assessed from the shapes extracted from the at least one 3D computed tomography image. In one embodiment of the method the evaluation of the shape of the femoral head is based on the sphericity of the femoral head. In another embodiment the evaluation of the shape of the acetabulum is based on the sphericity of the acetabulum. In yet another embodiment the sphericity should have a tolerance zone of at least 1 mm, or at least 2 mm, or at least 3 mm, or at least 4 mm for the patient to be eligible for the medical implant. Similar to the roundness, this means that the shape of e.g. the femoral head should fit between two spheres having a difference in radii equal to the tolerance. In another embodiment the sphericity should be at least 0.80 or at least 0.85, or at least 0.9, or at least 0.93, or at least 0.95, or at least 0.98 for the patient to be eligible for the medical implant.

Instead of or in addition to determining roundness or sphericity of the femoral head and/or acetabulum, the scan of the joint may in another embodiment also be used to fit a circle or sphere to the femoral head and/or acetabulum. Such a fit may be performed by selecting points belonging to the femoral head or acetabulum and use for example the method of least squared to fit a circle or sphere to the selected points. Selecting the points belonging to the femoral head or acetabulum is preferably automatic. In another embodiment the points may be selected manually. In yet another embodiment, automatically selected points may be adjusted manually by including and/or excluding and/or moving points. Customizing the implant may also be performed by fitting the smallest sphere that can include the entire femoral head or the largest sphere fitting inside the acetabulum.

When the implant is attached to the subject, the bone should preferably be shaped such that the implant cannot easily detach from the femoral head. Therefore, in one embodiment the radius of the femoral neck below the femoral head should be at least 1 mm, or at least 1.5 mm or at least 2 mm, or at least 2.5 mm lower than the radius of the femoral head for the patient to be eligible for the medical implant. This will keep the implant attached to the femoral head. However, it is still preferred that the implant is at least initially unconstrained after attachment to the femoral head. Soft tissue may then develop at the implant which may lead to the implant becoming attached to the femoral head or the acetabulum. In a further embodiment of the disclosure, the thickness at the top of the shell tₜ and the thickness at the equatorial line tₛ are selected based on measurements of the length of both legs of the subject. This is used to determine leg length discrepancy of the subject such that this measure may be used when customizing the implant. The thickness of the implant may then be used for reducing or correcting leg length discrepancy when possible.

The present disclosure is furthermore related to a decision support system for assessing eligibility of a subject for the customized medical implant and/or for selecting the parameters for the implant. The system however does not form part of the present invention. This system is based on at least one 3D computed tomography image of the diseased joint. The system further comprises a processing unit configured for extracting the shapes of the femoral head and acetabulum from said at least one 3D computed tomography image and for evaluating the shapes of the femoral head and acetabulum to assess subject eligibility. In one embodiment the processing unit is further configured for determining the roundness of the femoral head and/or acetabulum in at least one cross-sectional scan of a hip joint of said subject. In another embodiment the processing unit is further configured for determining the sphericity of the femoral head and/or acetabulum in the at least one 3D computed tomography image of a hip joint of said subject. In yet another embodiment the processing unit is further configured for determining the degree of narrowing at the femoral neck compared with the femoral head.

The decision support system may further comprise a non-transitive, computer-readable storage device for storing instructions that, when executed by a processor, performs a method for assessing eligibility of a subject for the customized medical implant and/or for selecting the parameters for the implant as herein described. The system may comprise a mobile device comprising a processor and a memory and being adapted to perform the method but it can also be a stationary system or a system operating from a centralized location, and/or a remote system, involving e.g. cloud computing. The disclosure further relates to a computer program having instructions which when executed by a computing device or system cause the computing device or system to identify an unauthorized access of an account of an online service according to the described method. Computer program in this context shall be construed broadly and include e.g. programs to be run on a PC or software designed to run on smartphones, tablet computers or other mobile devices.

In order to more carefully screen and select patients eligible for the customized medical implant, the decision support system is in one embodiment configured to include certain criteria for the patient. Criteria for a patient to be eligible for the implant may include patients with clinical complaints with unilateral or bilateral hip osteoarthritis with a preserved roundness of the femoral head, where conservative treatment has become unsuccessful and insufficient. Criteria excluding a patient from eligibility may be selected from the group of: secondary osteoarthritis following congenital hip dislocation, Calve-Legg-Perthes disease, infectious hip joint disease with deformed femoral head, moderate to severe hip dysplasia, hip fracture surgery with pinning or dynamic hip screw or intramedullary nails with hip screw, slipped femoral capital epiphysiolysis, acetabular fractures, aseptic femoral head necrosis and dysbaric osteonecrosis.

The decision support system is preferably configured for segmentation of the different bones in the scan such that the surfaces of the bones may be analyzed in order to customize the implant. The segmentation of the bone may be carried out manually or automatically by a computer or a combination where a computer provides a suggestion for the segmentation which is subsequently adjusted manually. In one embodiment the extraction of the shapes of the femoral head and acetabulum are based on at least one intensity threshold for distinguishing at least the cortical bone from the rest of the tissue. The threshold of the scan may be adjusted such that it identifies the rapid change in values of the scan at the edge of the cortical bone. This threshold value may be set manually or automatically. The segmentation process is preferably automatic. However, it may happen that software for automatic segmentation of the scan will not yield good results. In such cases it may be necessary to adjust the segmentation manually. Such cases may be when the cortical bone is thin or when the cortical bone surface of two bones are only separated by a small distance.

A model for automatic segmentation can be constructed in various ways. One method is to use a reference model for segmentation of subsequent scans. Therefore, in another embodiment the extraction of the shapes of the femoral head and acetabulum is based on a reference scan or a reference model such that the segmentation of the reference scan can be deformed or transformed to the scan of the patient, thereby acting as a reference for segmentation of a scan.

In another embodiment a model for automatic segmentation may be based on a large dataset. This can be achieved by having a number of scans that have been manually adjusted to obtain good segmentation of the bones. This dataset of segmented scans may then be used for the model to learn anatomic variations between subjects and use this to better identify and segment bones in new scans. This may provide better automatic segmentation than other models.

The at least one 3D computed tomography image will consist of a number of voxels giving the image a given resolution. Because of the finite, and in some cases limited, number of voxels, the image may appear rough containing many steps and edges. In one embodiment the image is smoothened by a surface reconstruction algorithm. In one embodiment the marching cubes algorithm is applied to provide a polygonal mesh giving the model a smoother surface. In another embodiment a surface reconstruction algorithm, such as the Markov ransom field surface reconstruction algorithm, is applied to provide an even smoother surface than the polygonal mesh.

### Detailed description of the drawings

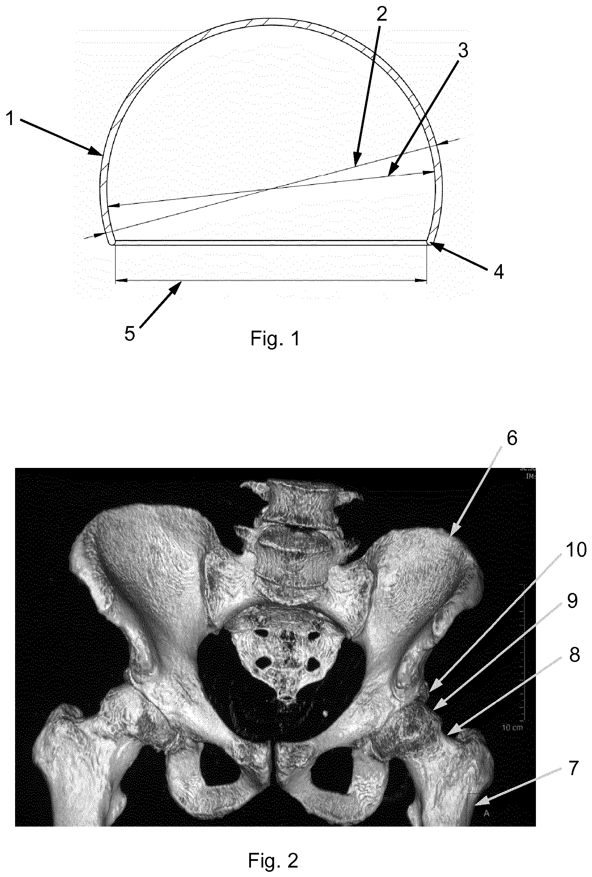

**Fig. 1** shows a cross-sectional view of one embodiment of the customized medical implant **1** for relieving pain in a diseased hip joint. The dome-shaped shell **1** is in this embodiment spherically shaped. The implant has an orifice **5** at the bottom of the shell such that the shell can be mounted on the femoral head of a patient. The implant is furthermore characterized by an outer shell radius **2** and an inner shell radius **3.** The edge **4** at the orifice is preferably rounded.

**Fig. 2** is an example of a 3D computed tomography image of a patient showing both hip joints. The top part of the femoral bone **7** is seen at the bottom of the image along with the femoral neck **8** and femoral head **9.** The entire pelvis **6** is visible in the image including the outer edge of the acetabulum **10** where the femoral head **9** connects to the pelvis **6.**

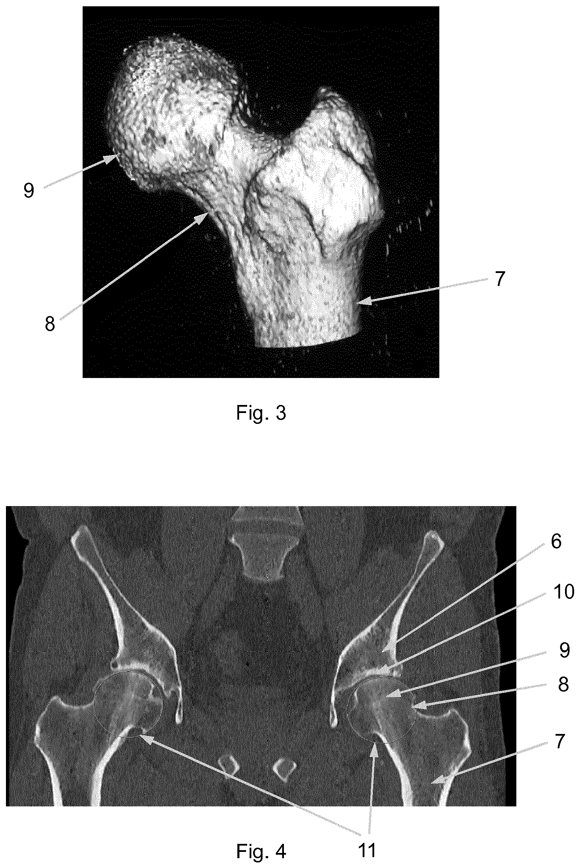

**Fig. 3** is an example of the femoral bone part isolated from a 3D computed tomography image of a hip joint. The femoral bone **7** together with the femoral neck **8** and the femoral head **9** are shown in the image. Separating the femoral bone part from the pelvic bone part, i.e. the acetabulum, is useful when determining the optimum parameters for the customized medical implant.

**Fig. 4** shows an example of a 2D X-ray image anterior-posterior projection of both hip joints. The image is based on a computerized tomography scanning. The image shows the femoral bone **7,** femoral neck **8,** femoral head **9,** acetabulum **10** and part of the pelvic bone **6.** In the image both femoral heads are encircled **11** by digital means. This is used to determine the diameter of the customized implant.

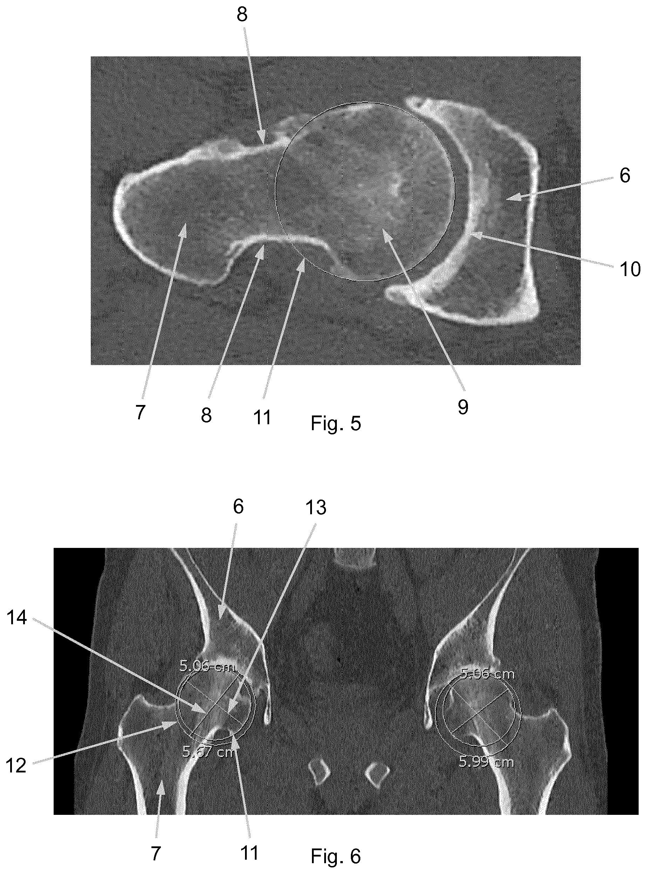

**Fig. 5** shows an example of a 2D X-ray horizontal plane of the right hip joint of a patient. Again, the femoral bone **7,** femoral neck **8,** femoral head **9,** acetabulum **10** and part of the pelvic bone **6** are shown in the image. Such images may be used to determine parameters for the customized implant and for determining eligibility for the surgery. In this case the femoral head **9** is well preserved and has a high degree of roundness. Again, the femoral head **9** is encircled **11** by digital means. The image furthermore shows clear separation between the femoral head **9** and acetabulum **10** of the hip joint. In cases with less separation, traction to the leg may be necessary for sufficient separation between the femoral head **9** and the acetabulum **10.**

**Fig. 6** is an example of a CT scan of a patient showing both hip joints in anterior-posterior plane. Both hips show radiological signs of osteoarthritis with subchondral bone sclerosis and rim osteophytes. The femoral head and acetabulum are clearly separated for both hips. The femoral head of each hip is encircled **11** by digital means. The diameter **13** of this circle is also shown. Similarly, the acetabulum of each hip is encircled **12** by digital means and again the diameter **14** is shown. The diameters of the femoral head and acetabulum may be used as an indicator for the thickness of the potential implant.

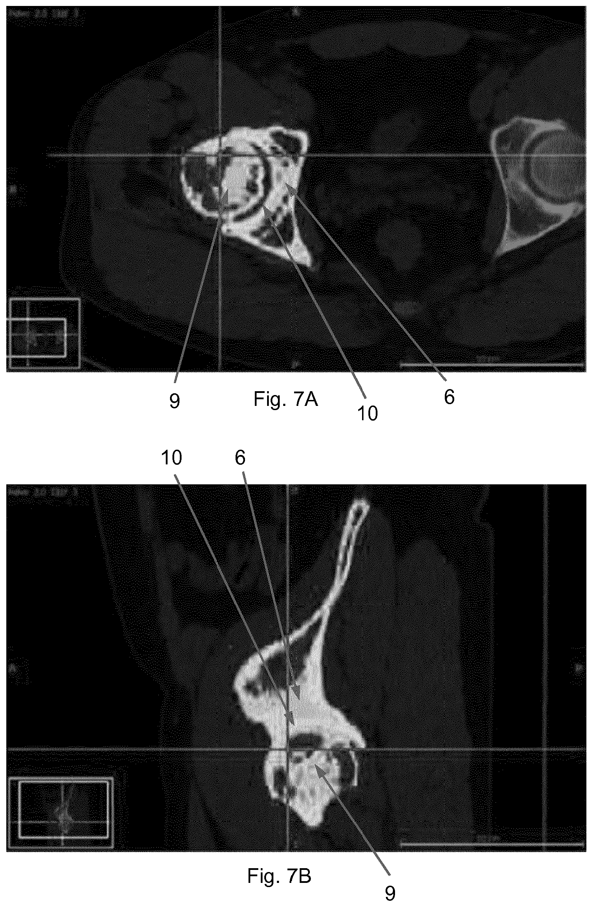

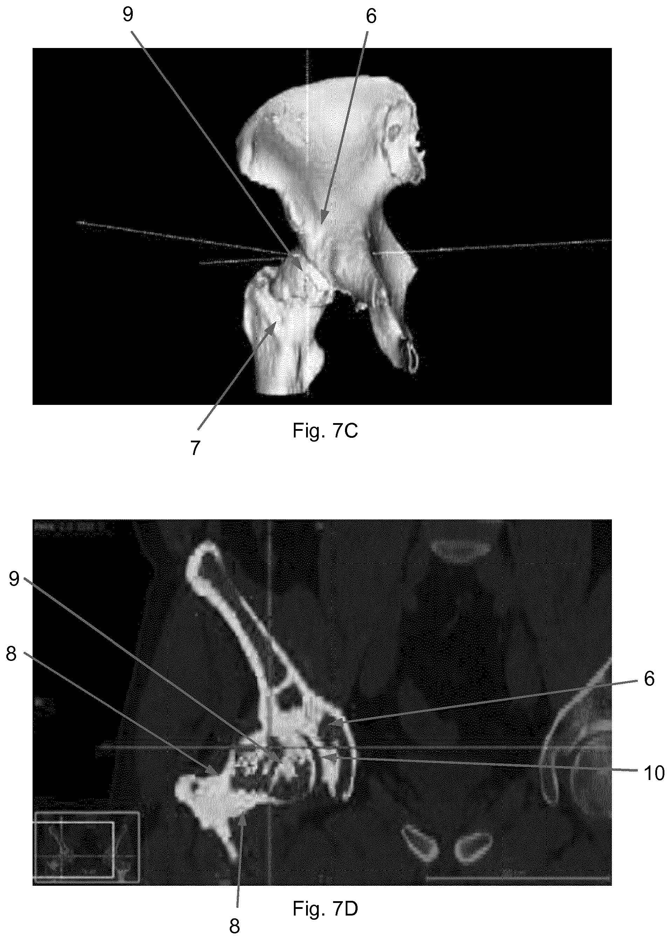

**Figs. 7A-D** show screenshots from ITK-Snap used in one embodiment of the disclosure. The program is used for segmentation of the scan and identification of bones and surfaces. For this embodiment only the program with its built-in functions was used for segmentation. In Fig. 7A the highlighted areas show the segmented bones with the femoral head **9** and the pelvic bone **6** clearly separated. Fig. 7B shows a different angle with less clear separation between the femoral head **9** and acetabulum **10.** Figs. 7C and 7D each show other angles of the segmented bones for this embodiment.

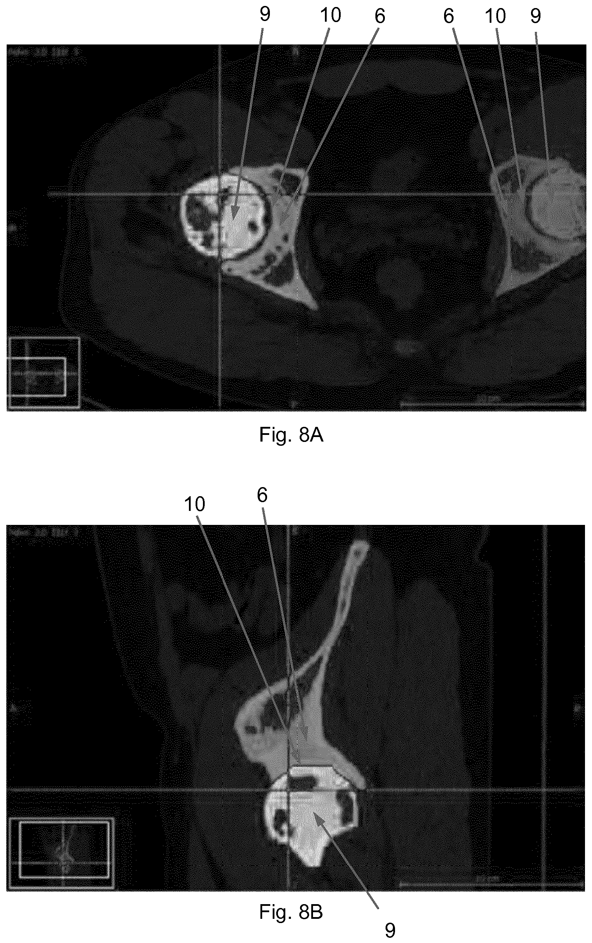

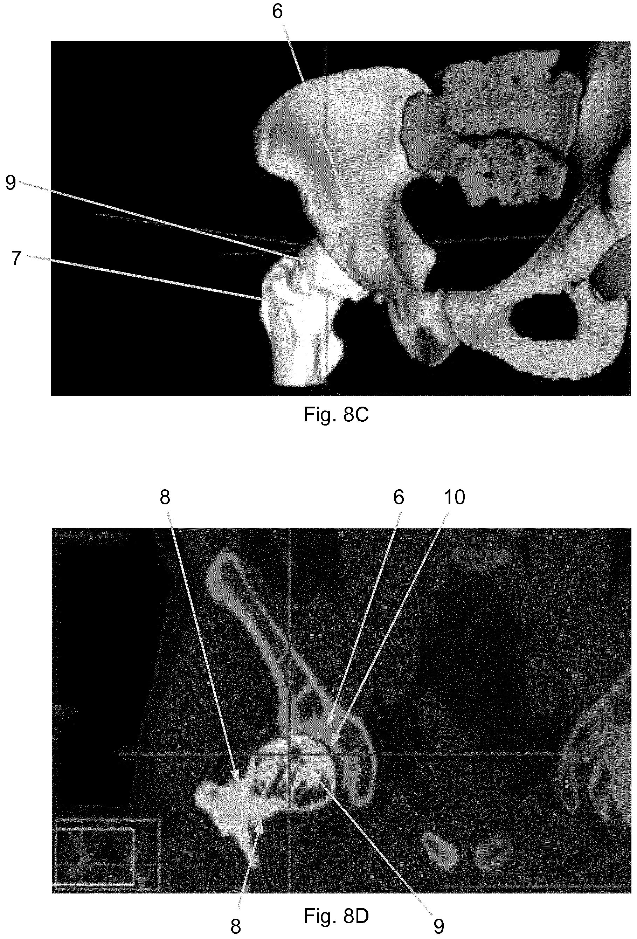

**Figs. 8A-D** are screenshots from ITK-Snap used in another embodiment for segmentation of the scan and identification of bones and surfaces. In this embodiment some points have been created and/or adjusted manually to create a better model and better segmentation of the joint. It may sometimes be necessary to adjust and/or create and/or delete and/or move some points of the segmented bones manually to obtain a better and/or smoother and/or more correct model of the joint. Fig. 8A shows the same scan as Fig. 7A, but now with better segmentation due to manual adjustment such that each bone is identified and highlighted in different grayscales. Figs. 8B-D show similar views of the joint as Figs. 7B-D of the manually adjusted segmentation with different bones highlighted in different grayscales.

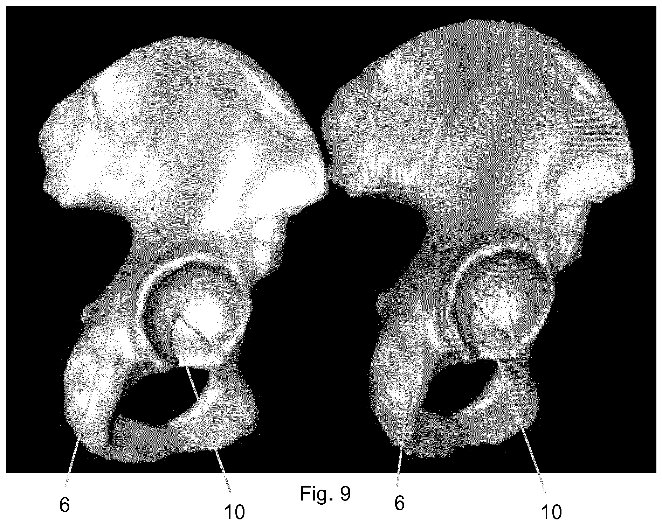

**Fig. 9** shows a surface representation of the pelvic bone from a scan. The raw scan shown on the right may contain edges and steps because of the resolution of the scan. The rough surface may be smoothened using various techniques. One method shown in the embodiment to the left is to use a marching cubes algorithm together with a surface reconstruction algorithm to yield a higher quality surface of the bone for customizing the implant.

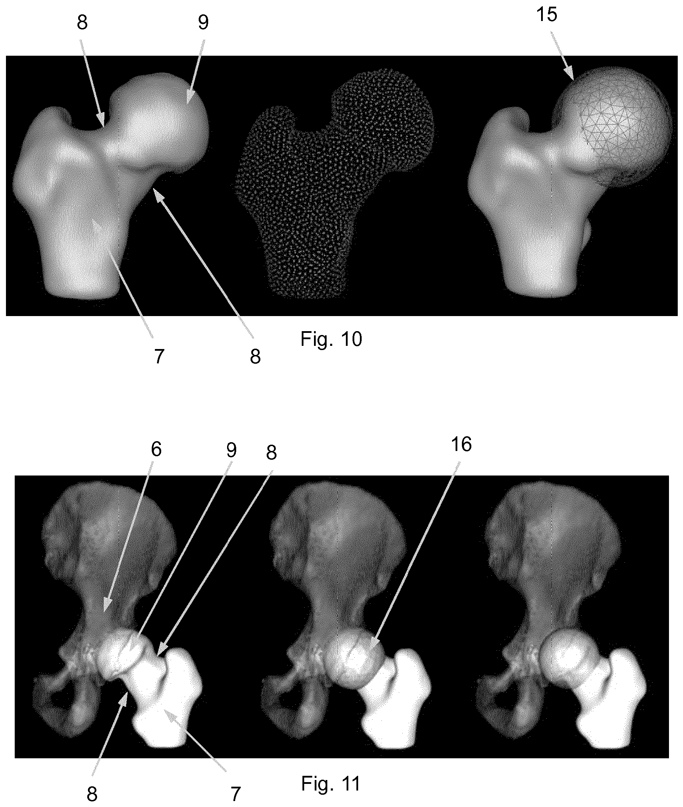

**Fig. 10** shows is an illustration of the scanned and smoothened femoral bone. Points on the surface are shown in the middle part of the figure. In this embodiment the points belonging to the femoral head have been manually selected. This process could also be automatic, or an automatic selection of points could be suggested and then manually corrected if needed. The points belonging to the femoral head are used to fit a sphere to the femoral head (right). This is used to determine the radius of said sphere which is used to determine the inner equatorial shell radius of the implant. The sphere may be fitted using the method of least squares. The sphere may also be fitted to determine the smallest sphere fitting on the outside of the femoral head.

**Fig. 11** shows the scanned bones of a hip joint and an implant according to one embodiment of the disclosure. The left part shows the segmented bones as obtained from the scan with subsequently smoothened surfaces. An implant calculated and customized to fit the specific joint is shown inserted into the joint in the middle part of the figure. The right part shows of the figure shows a smoothened and slightly transparent version of the implant inserted in the joint.

## Claims

1. A customized medical implant (1) for attachment to and at least partly covering the natural femoral head (9) of a hip joint of a subject, said medical implant comprising a dome shaped shell (1) having a height h, an equatorial line, an equatorial plane, an inner equatorial shell radius rₛ, an orifice (5), an orifice radius rₒ, a thickness tₛ at the equatorial line, and a thickness tₜ at the top of the dome, wherein one or more of the thickness tₛ, the thickness tₜ, the equatorial shell radius rₛ, the orifice radius rₒ and the shell height h are customized to the hip joint of the subject based on at least one 3D computed tomography image showing substantially the entirety of the natural femoral head (9) and the acetabulum (10) of said hip joint, wherein the shell (1) comprises an inner surface that is spherical at least above the equatorial plane and an outer surface that is spherical at least above the equatorial plane, wherein the spherical inner surface and the spherical outer surface define spherical centers, **characterised in that** the spherical center of the inner surface is closer to the orifice (5) than the spherical center of the outer surface, so that the thickness at the top of the dome is larger than the thickness at the equatorial line, tₜ > tₛ.

## Patentansprüche

1. Maßgefertigtes medizinisches Implantat (1) zur Befestigung an und zum zumindest teilweisen Bedecken des natürlichen Femurkopfes (9) eines Hüftgelenks eines Subjekts, wobei das medizinische Implantat eine kuppelförmige Schale (1) mit einer Höhe h, einer Äquatorlinie, einer Äquatorebene, einem inneren äquatorialen Schalenradius rₛ, einer Öffnung (5), einem Öffnungsradius r₀, einer Dicke tₛ an der Äquatorlinie und einer Dicke tₜ an der Spitze der Kuppel umfasst, wobei eine oder mehrere der Dicke tₛ, der Dicke tₜ, des äquatorialen Schalenradius rₛ, des Öffnungsradius rₒ und der Schalenhöhe h für das Hüftgelenk des Subjekts basierend auf mindestens einem 3D-Computertomographie-Bild, das im Wesentlichen die Gesamtheit des natürlichen Femurkopfes (9) und des Acetabulums (10) des genannten Hüftgelenks zeigt, angepasst sind, wobei die Schale (1) eine innere Oberfläche, die zumindest oberhalb der Äquatorebene sphärisch ist, und eine äußere Oberfläche, die zumindest oberhalb der Äquatorebene sphärisch ist umfasst, wobei die sphärische innere Oberfläche und die sphärische äußere Oberfläche sphärische Zentren definieren, **dadurch gekennzeichnet, dass** das sphärische Zentrum der inneren Oberfläche näher an der Öffnung (5) ist als das sphärische Zentrum der äußeren Oberfläche, so dass die Dicke an der Spitze der Kuppel größer ist als die Dicke an der Äquatorlinie, tₜ > tₛ.

## Revendications

1. Dispositif médical personnalisé (1) pour fixation à et recouvrement au moins partiel de la tête fémorale naturelle (9) d'une articulation de la hanche d'un sujet, ledit dispositif médical comprenant une coque en forme de dôme (1) ayant une hauteur h, une ligne équatoriale, un plan équatorial, un rayon de coque équatorial interne rₛ, un orifice (5), un rayon d'orifice rₒ, une épaisseur tₛ à la ligne équatoriale, et une épaisseur tₜ au sommet du dôme, où un ou plusieurs de l'épaisseurs tₛ, l'épaisseur tₜ, le rayon de coque équatorial rₛ, le rayon d'orifice rₒ et la hauteur de coque h sont personnalisés à l'articulation de la hanche du sujet sur la base d'au moins une image 3D de tomographie assistée par ordinateur montrant sensiblement l'intégralité de la tête fémorale naturelle (9) et de l'acétabulum (10) de ladite articulation de la hanche, dans lequel la coque (1) comprend une surface interne qui est sphérique au moins au-dessus du plan équatorial et une surface externe qui est sphérique au moins au-dessus du plan équatorial, où la surface interne sphérique et la surface externe sphérique définissent des centres sphériques,
**caractérisé en ce que** le centre sphérique de la surface interne est plus proche de l'orifice (5) que le centre sphérique de la surface externe, de sorte que l'épaisseur au sommet du dôme est plus grande que l'épaisseur à la ligne équatoriale, tₜ > tₛ.
